# EUROPÄISCHE PATENTSCHRIFT

(11) **EP 1 393 714 B1**
(45) Veröffentlichungstag und Bekanntmachung des Hinweises auf die Patenterteilung: **21.11.2007**
(21) Anmeldenummer: 03014574.2
(22) Anmeldetag: 07.07.2003
(51) Int. Cl.: A61K 8/42, A61K 8/44, A61K 8/46, A61K 8/86, A61K 8/92, A61Q 5/02, A61Q 19/10

(54) **Reinigungszubereitung aus zwei Komponenten**
Cleaning composition comprising two components
Composition nettoyante comprenant deux éléments

(30) Priorität: 29.08.2002 DE 10239649
(43) Veröffentlichungstag der Anmeldung: 03.03.2004
(73) Patentinhaber: Beiersdorf AG, 20245 Hamburg (DE)
(72) Erfinder: Ruppert, Stephan Dr., 20259 Hamburg (DE); Counradi, Katrin, 22143 Hamburg (DE); Treu, Jens Dr., 22844 Norderstedt (DE); Rohde, Olaf, 22880 Wedel (DE)

(56) Entgegenhaltungen:
- EP-A- 0 691 127
- EP-A- 0 916 334
- US-A- 6 132 738

## Beschreibung

Die vorliegende Erfindung betrifft Kosmetika und/oder Dermatika aus zwei Komponenten

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Cremes, Salben oder Lotionen, dienen meist der Befeuchtung und Rückfettung der Haut, denn die Aufgabe der Hautpflege ist es, den durch das tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern. Häufig sind Hautpflegeprodukten Wirkstoffe zugesetzt, welche die Haut regenerieren und beispielsweise ihre vorzeitige Alterung (z.B. das Entstehen von Fältchen, Falten) verhindern und vermindern sollen. Zum Schutz vor der schädlichen UV-Strahlung des Sonnenlichtes sind vielen kosmetischen und dermatologischen Produkten UV-Lichtschutzfilter zugesetzt.

Herkömmlicher Weise werden kosmetische Reinigungszubereitungen aus wässrigen Tensidlösungen hergestellt. Meist kommen dabei gelförmige Zubereitungen zum Einsatz. Diese haben den Vorteil, dass sie zum einen aufgrund Ihres Gehaltes an anionischen Tensiden meist sehr gut schäumen und zum anderen transparent sind, so dass sich eine Vielzahl interessanter Effektstoffe (z.B. Gasblasen, Glitterstoffen, Kügelchen, Farbschlieren, etc.) in die Formulierung einarbeiten lassen.

Der Nachteil an derartigen Zubereitungen besteht in dem Umstand, dass die Formulierungen bei der Anwendung die Haut und Hautanhangsgebilde stark entfetten. Um den Fettverlust insbesondere der Haut zu kompensieren, wurden in jüngerer Zeit sogenannte Duschöle und Duschemulsionen entwickelt, die einen mehr oder weniger großen Lipidanteil aufweisen und damit rückfettend wirken. Diese Zubereitungen schäumen allerdings nur sehr schlecht, da man den Formulierungen keine stark schäumenden anionischen Tenside zusetzen kann. Ferner lassen sich in solche Zubereitungen meist keine Effektstoffe oder andere optische Effekte einarbeiten, da Emulsionen in der Regel milchig-trübe sind und sich, ebenso wie in Duschölen, die Effektstoffe nicht ausreichend stabilisieren fassen. Damit sind solche Produkte für den Verbraucher optisch und sensorisch relativ unattraktiv.

Es war daher die Aufgabe der vorliegenden Erfindung, die Mängel des Standes der Technik zu beseitigen oder zumindest zu lindern und stark rückfettende kosmetische und/oder dermatologische Reinigungszubereitungen zu entwickeln, die eine für den Verbraucher interessante Schaumcharakteristik aufweisen d.h. stark aufschäumen und einen cremigen feinblasigen Schaum bilden. Zusätzlich sollte die Zubereitung so formuliert werden, dass sich für den Anwender optisch interessante Effekte erzeugen und/oder einarbeiten lassen.

Überraschend gelöst wird die Aufgabe durch ein Kosmetikum und/oder Dermatikum enthaltend
a) eine transparente kosmetische und/oder dermatologische Reinigungszubereitung A in einer Menge von 25 bis 75 Gewichts-% der Gesamtzubereitung,
b) eine transparente Zubereitung B aus einer oder mehreren öllöslichen Verbindungen und mindestens einem öllöslichen Tensid in einer Menge von 25 bis 75 Gewichts-% der Gesamtzubereitung,
dadurch gekennzeichnet, dass die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffung austreten, oder zwei getrennten Öffnungen entnommen werden, sowie dadurch gekennzeichnet, dass in der zubereitung B ein oder mehrere öllösliche Tenside im einer gesamtkonzentration von 1-10 Gew.-%, bezogen auf das besamtgewicht von B. eingesetzt werden.

Das erfindungsgemäße Kosmetikum beziehungsweise Dermatikum zeigt eine hervorragende Schaumcharakteristik. Darüber hinaus lassen sich nicht nur mit Effektstoffen erfindungsgemäß besonders attraktive optische und/oder sensorische Effekte erzielen. Schon das Verreiben zweier für sich genommen klarer (transparenter) Zubereitungen A und B führt erfindungsgemäß zu einer trüb-weißen Gesamtzubereitung und stellt damit einen reizvollen optischen Effekt dar.

Zwar beschreiben auch die WO 96/02230, die WO 96/37420, die WO 98/33477 sowie die EP 0916334 tensidhaltige Zubereitungen in Doppelkammersystemen, doch konnten diese Schriften nicht den Weg zur vorliegenden Erfindung weisen. Auch die Offenbarungen der EP 0916334, EP 0691127 und US 6132738 konnten nicht den Weg zur vorliegenden Erfindung weisen.

Das erfindungsgemäße Kosmetikum und/oder Dermatikum enthält in Zubereitung B ein oder mehrere öllösliche Tenside in einer Gesamtkonzentratrion von 1 bis 10 Gewichts-%, bevorzugt von 3 bis 6 Gewichts-% und ganz besonders vorteilhaft von bis Gewichts-%, jeweils bezogen auf das Gesamtgewicht der Zubereitung B.

Es ist erfindungsgemäß von Vorteil, wenn das erfindungsgemäße Kosmetikum und/oder Dermatikum als öllösliche Tenside in der Zubereitung B Alkylethersulfate mit Triisopropanolamin, Diisopropanolamin und/oder Monoisopropanolamin als Kation eingesetzt werden. Diese können erfindungsgemäß vorteilhaft kombiniert werden mit Ethylenglykolethern der Struktur

CH₃(CH₂)₁₀CH₂(OCH₂CH₂)ₙOH,

welche unter der INCI-Bezeichnung Laureth-n im Handel erhältlich sind, sowie Fettsäureamidderivaten der Struktur

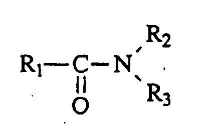

Erfindungsgemäß bevorzugte Alkylethersulfate sind dabei Laurylethersulfate, insbesondere Triisopropanolaminlaurylethersulfat (INCI: TIPA-Laureth Sulfate) und/oder Monoisopropanolaminlaurylethersulfat (INCI: MIPA-Laurethsulfat).

Der erfindungsgemäß bevorzugte Ethylenglykolether ist das nach der INCI bezeichnete Laureth-4.

Erfindungsgemäß bevorzugte Fettsäureamidderivate Stellen die Cocosfettsäureamid-derivate, insbesondere Cocosfettsäurediethanolamid (INCI: Cocamide DEA) dar.

Die erfindungsgemäße Zubereitung B ist erfindungsgemäß vorteilhaft wasserfrei. Dabei bedeutet "wasserfrei" im Sinne der vorliegenden Erfindung, dass die Zubereitung B eine maximale Wasserkonzentration von höchstens 1 Gewichts-%, bezogen auf das Gesamtgewicht der Zubereitung B aufweist.

Es ist erfindungsgemäß vorteilhaft, wenn als Zubereitung A eine tensidhaltige Reinigungszubereitung eingesetzt wird.

Die erfindungsgemäße Zubereitung A kann vorteilhafter Weise ein oder mehrere Tenside enthalten. Erfindungsgemäß vorteilhaft können sowohl anionische, kationische, nichtionische und zwitterionische Tenside eingesetzt werden. Erfindungsgemäß bevorzugt ist insbesondere der Einsatz von einem oder mehreren anionischen Tensiden in der Zubereitung A.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat,Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat; Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Atuminiumstearat, Magnesiumatkanotat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders vorteilhaft, wenn als Tenside in der Zubereitung A Natriumlaurethsulfat, Natriummyrethsulfat, Cocamidopropylbetain, Laurylglucosid, Natriumcocoylglutamat und/oder Natriumcocoamphoacetat eingesetzt werden.

Erfindungsgemäß bevorzugt sind Tensidkombinationen aus Alkylethersulfaten mit amphoteren Cotensiden, wobei Tensidkombinationen aus Natriumlaurethsulfat und Cocamidopropylbetain oder Natriumlaurethsulfat, Cocamidopropylbetain und Natriumcocoylglutamat einerseits sowie Tensidkombinationen aus Natriummyrethsulfat und Decylglycosid andererseits besonders bevorzugt ist. Insbesondere mit diesen Tensidkombinationen lassen sich Reinigungszubereitungen mit hoher Schaumleistung, sowie besonders cremigem Schaum bei der Anwendung herstellen.

Es ist vorteilhaft im Sinn der vorliegenden Erfindung, wenn der Gehalt an einem oder mehreren waschaktiven Tensiden in der kosmetischen Zubereitung A aus dem Bereich von 1 bis 30 Gewichts-%, bevorzugt von 5 bis 25 Gew.-%, ganz besonders vorteilhaft von 10 bis 20 Gew.-% gewählt wird, jeweils bezogen auf das Gesamtgewicht der Zubereitung

Erfindungsgemäß vorteilhaft kann mindestens eine der erfindungsgemäßen Zubereitungen Polysorbate enthalten.

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmoriooleat (Tween 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der jeweiligen Zubereitung einzeln oder als Mischung mehrer Polysorbate, eingesetzt.

Die erfindungsgemäße Zubereitung A kann als wässrige Lösung oder wässrige Phase einer Emulsion neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Die kosmetische Zubereitung A kann zusätzlich neben einer oder mehreren Wasserphasen eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion) sein, wobei transparente oder transluzente Mikroemulsionen erfindungsgemäß besonders bevorzugt sind.

Die erfindungsgemäßen Zubereitungen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9)

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der jeweiligen Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der jeweiligen Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der jeweiligen Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel. Auch die Vitamin D₃-analoga Tacalcitol, Calcipotriol, Tacalcitol, Colecalciferol sowie Calcitrol (Vitamin D₃) und/oder Fumarsäureester können erfolgreich in die Zubereitungen hineinformuliert werten.

Die Ölphase bzw. Ölphasen der erfindungsgemäßen Formulierungen wird/werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethyl-hexylpalmitat, .2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann eine Ölphase vorteilhaft gewählt werden aus der Gruppe der Dialkylether und Dialkylcarbonate, vorteilhaft sind z.B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Corapan®TQ von Haarmann & Reimer*)*.*

Auch beliebige Abmischungen solcher Öl- und Wachskomponenten sind vorteilhaft im Sinne der vorliegenden Erfindung einzusetzen.

Ferner kann eine Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann eine Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene-Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß besonders vorteilhafte Ausführungsformen des erfindungsgemäßen Kosmetikums und/oder Dermatikums sind dadurch gekennzeichnet, dass als öllösliche Verbindungen in der Zubereitung B Sojaöl, Rizinusöl, Sonnenblumenöl, Weizenkeimöl, und/oder Macadamianussöl eingesetzt werden.

Erfindungsgemäß bevorzugt sind damit Kosmetika bzw. Dermatika, deren Zubereitung A eine wässrige Lösung oder ein Gel darstellen und deren Zubereitung B eine mit mindestens einem öllöslichem Tensid versetzte Lipidphase darstellt, die besonders bevorzugt wasserfrei ist.

Die erfindungsgemäßen Zubereitungen können auch alle nach der Kosmetikverordnung zugelassenen wasserlöslichen und/oder öllöslichen UV-A-, UV-B- und/oder Breitbandfiltersubstanzen enthalten.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, weitere Pflanzenextrakte, Vitamine, Wirkstoffe, Konservierungsmittel, Bakterizide, Repellentien, Selbstbräuner, Depigmentierungsmittel, Pigmente, die eine färbende Wirkung haben, weichmachende, anfeuchtende und/oder feuchthaltende Substanzen, oder andere übliche Bestandteile einer kosmetischen oder dermatologischen Formulierung wie Emulgatoren, Polymere, Schaumstabilisatoren und Elektrolyte.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E-231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten.

Erfindungsgemäß bevorzugt befinden sich ein oder mehrere Konservierungsmittel in der Zubereitung A.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische inhaltsstoffe (INCl) als Polyquaternium benannten Verbindungen dar (insbesondere Polyquaternium-1 bis Polyquaternium-56).

Es ist erfindungsgemäß vorteilhaft, den mindestens einer der erfindungsgemäßen Zubereitungen Glittersoffe und/oder andere Effektstoffe zuzusetzen.

Auch ist es vorteilhaft im Sinne der vorliegenden Erfindung, wenn mindestens eine der beiden Zubereitungen A und B ein oder mehrere Hydrokolloide in einer Konzentration von 0,01 bis 10 Gewichts-%, bevorzugt in einer Konzentration von 0,1 bis 7 Gewichts-% und ganz besonders bevorzugt in einer Konzentration von 0,3 bis 5 Gewichts-%, jeweils bezogen auf das Gesamtgewicht der Zubereitung enthält.

Als erfindungsgemäß vorteilhafte Hydrokolloide.werden Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Celluloseether, Hydroxyethyl- und -propylcellulosederivate, Polysaccharide, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 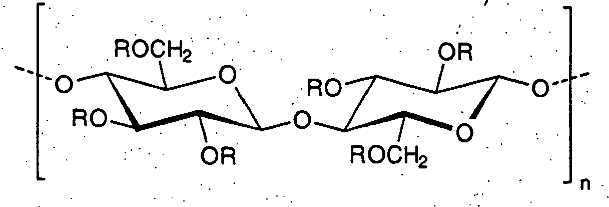 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure. Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt.von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute 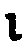 -Carrageenan ist sowohl wasserlöslich als auch gelbildend. Weitere Carrageen-Typen werden ebenfalls mit griechischen Buchstaben bezeichnet: α, β, y, µ, ν, ξ, π, ω, χ. Auch die Art vorhandener Kationen (K+, NH4+, Na+, Mg2+, Ca2+) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

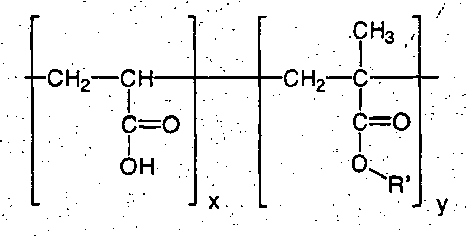

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCl-Bezeichnung"Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCl-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCl-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Erfindungsgemäß bevorzugt ist es insbesondere, neutralisierte oder teilneutralisierte Polyacrylate (z.B. Carbopole der Firma Noveon) einzusetzen.

Die Gesamtmenge an einem oder mehreren Hydrokolloiden wird vorteilhaft kleiner als 5 Gew.-%, bevorzugt zwischen 0,1 und 5 Gew.-%, bezogen auf das Gesamtgewicht der jeweiligen Zubereitung, gewählt.

Es ist vorteilhaft im Sinn der vorliegenden Erfindung, wenn der Gehalt an einem oder mehreren Polyacrylaten in einer kosmetischen oder dermatologischen Reinigungsemulsion aus dem Bereich von 0,1 bis 8 Gew.-%, ganz besonders vorteilhaft von 0,1 bis 5 Gew.-% gewählt wird, jeweils bezogen auf das Gesamtgewicht der jeweiligen Zubereitung.

Erfindungsgemäße Verpackungsbehältnisse können vorteilhaft aus einer Kunststoffflasche oder einer Aerosoldose bestehen. Erfindungsgemäß vorteilhaft kann auch mindestens eine der beiden Zubereitungen mit einem Treibmittel aufgeschäumt werden.

Als erfindungsgemäß vorteilhafte Verpackungsbehältnisse können beispielsweise Verpackungen gewählt werden, wie sie in der WO 96/37420 oder der GB 2 180 215 offenbart sind. Auch Verpackungssysteme wie sie von mehrfarbig gestreifter Zahnpasta her bekannt sind, lassen sich erfindungsgemäß vorteilhaft verwenden unter der Maßgabe, dass die beiden Zubereitungen durch eine undurchlässige Sperre voneinander getrennt sind und erst beim Austritt aus der Verpackung zu der bekannten streifenförmigen Gesamtzusammensetzung zusammengesetzt werden.

Vorteilhafte Verpackungsbehältnisse stellen ferner mit zwei Kammern versehene Quetschflaschen dar, die entweder für beide Zubereitungen eine gemeinsame Entnahmeöffnung oder aber zwei separate Entnahmeöffnungen aufweisen.

Erfindungsgemäß vorteilhafte Verpackungsbehältnisse stellen auch Doppelkammertuben mit ein oder zwei Entnahmeöffnungen dar.

Nicht zuletzt ist es erfindungsgemäß vorteilhaft, wenn beide Zubereitungen zusammen oder getrennt mit Hilfe eines Pump-und/oder Dosierspenders dem Verpackungsbehältnis entnommen werden können.

Generell bevorzugt sind Verpackungsbehältnisse, bei denen die beiden erfindungsgemäßen Zubereitungen A und B dem Behältnis aus zwei unterschiedlichen Entnahmeöffnungen entnommen werden.

Es ist erfindungsgemäß vorteilhaft, wenn die beiden Zubereitungen A und B unterschiedlich gefärbt und/oder mit unterschiedlichen Effektstoffen beladen sind.

Erfindungsgemäß ist die Verwendung des erfindungsgemäßen Kosmetikums und/oder Dermatikums zur Reinigung der Haut und/oder Hautanhangsgebilde.

Insbesondere ist die Verwendung des erfindungsgemäßen Kosmetikums und/oder Dermatikums als Duschgel, Wannenbad oder Haarwaschmittel (Haarshampoo) erfindungsgemäß.

Ferner eignen sich die erfindungsgemäßen Produkte hervorragend zur Reinigung von Gegenständen des täglichen Lebens (z.B. Geschirr, Tisch- und Schrankflächen, Autos).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

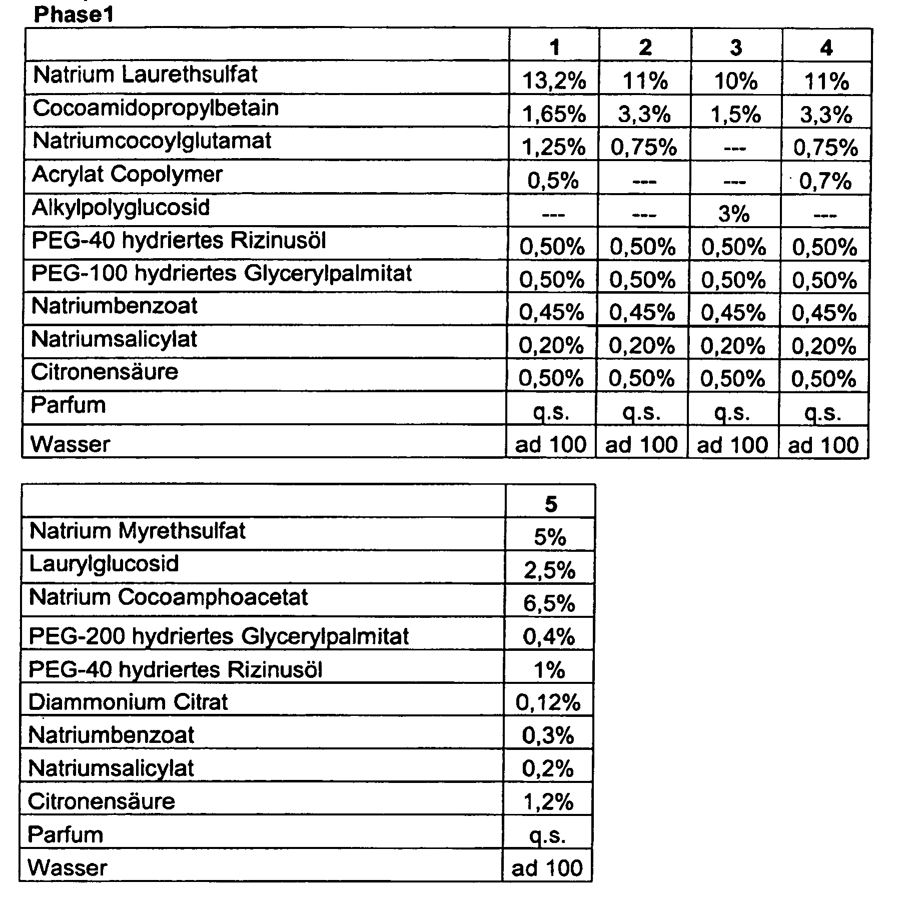

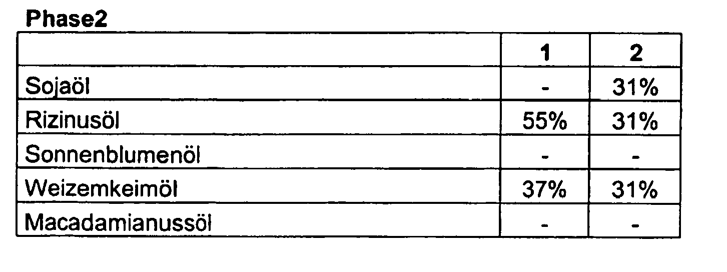

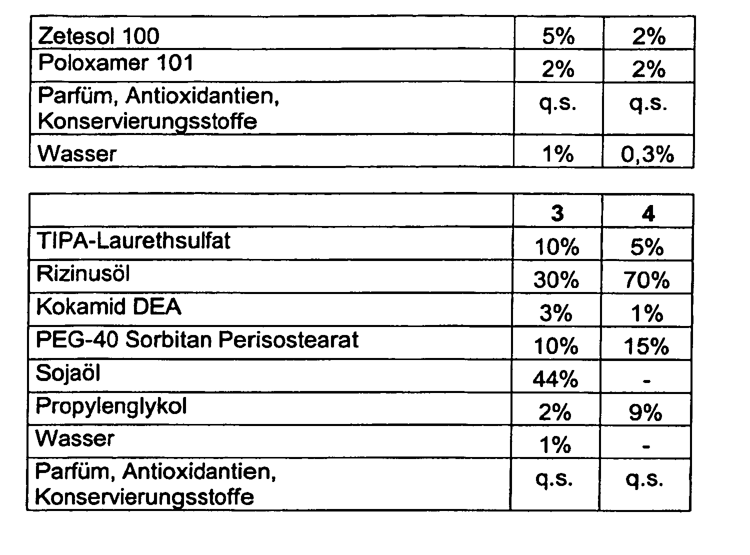

## Patentansprüche

1. Kosmetikum und/oder Dermatikum enthaltend
a) eine transparente kosmetische und/oder dermatologische Reinigungszubereitung A in einer Menge 25 von bis 75 Gewichts-% der Gesamtzubereitung,
b) eine transparente Zubereitung B aus einer oder mehreren öllöslichen Verbindungen und mindestens einem öllöslichen Tensid in einer Menge von 25 bis 75 Gewichts-% der Gesamtzubereitung,
**dadurch gekennzeichnet, dass** die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden sowie **dadurch gekennzeichnet, dass** in Zubereitung B ein oder mehrere öllösliche Tenside in einer Gesamtkonzentration von 1 bis 10 Gewichts-%, bezogen auf das Gesamtgewicht der Zubereitung B, eingesetzt werden.

2. Kosmetikum und/oder Dermatikum nach Anspruch 1, **dadurch gekennzeichnet, dass** als öllösliche Tenside in der Zubereitung B TIPA-Laurethsulfat, Kokamid DEA und PEG-40 Sorbitan Perisostearat eingesetzt werden.

3. Kosmetikum und/oder Dermatikum nach einem der Ansprüche 1 bis 2, **dadurch gekennzeichnet, dass** Zubereitung A anionische, kationische, amphotere und/oder nichtionische Tenside in einer Gesamtkonzentration von 1 bis 30 Gewichts-%, bezogen auf das Gesamtgewicht der Zubereitung A, enthält.

4. Kosmetikum und/oder Dermatikum nach einem der Ansprüche 1 bis 3, **dadurch gekennzeichnet, dass** in der Zubereitung A Natriumlaurethsulfat, Natriummyrethsulfat, Cocamidopropylbetain, Laurylglucosid, Natriumcocoylglutamat und/oder Natriumcocoamphoacetat eingesetzt werden.

5. Kosmetikum und/oder Dermatikum nach einem der Ansprüche 1 bis 4, **dadurch gekennzeichnet, dass** als öllösliche Verbindungen in der Zubereitung B . Sojaöl, Rizinusöl, Sonnenblumenöl, Weizemkeimöl, Macadamianussöl eingesetzt werden.

6. Verwendung eines Kosmetikums und/oder Dermatikums nach einem der vorhergehenden Ansprüche zur Reinigung der Haut und/oder Hautanhangsgebilde.

7. Verwendung eines Kosmetikums und/oder Dermatikums nach einem der vorhergehenden Ansprüche als Duschgel, Wannenbad oder Haarwaschmittel.

## Claims

1. Cosmetic and/or dermatological composition containing
a.) a transparent cosmetic and/or dermatological cleaning preparation A in a quantity of 25 to 75 % by weight of the total preparation.
b.) a transparent preparation B comprising one or more oil-soluble compounds and at least one oil-soluble surfactant in a quantity of 25 to 75 % by weight of the total preparation,
wherein the two preparations A and B are contained in separate compartments of a package and, in use, (a) they are delivered simultaneously through a common opening and are mixed before delivery, or they are discharged from the opening in the form of a strip, or (b) they are delivered through separate openings, and
wherein one or more oil-soluble surfactants are used in a total concentration of 1 to 10 % by weight, in relation to the total weight of preparation □.

2. Cosmetic and/or dermatological composition according to claim 1, wherein TIPA laureth sulfate, cocamide DEA and PEG-40 sorbitan perisostearate are used as oil-soluble surfactants in preparation B.

3. Cosmetic and/or dermatological composition according to one of claims 1 to 2, wherein preparation A contains anionic, cationic, amphoteric und/or nonionic surfactants in a total concentration of 1 to 30 % by weight, in relation to the total weight of preparation A.

4. Cosmetic and/or dermatological composition according to one of claims 1 to 3, wherein A sodium laureth sulfate, sodium myreth sulfate, cocamidopropyl betaine, lauryl glucoside, sodium cocoyl glutamate and/or sodium cocoamphoacetate are used in preparation A.

5. Cosmetic and/or dermatological composition according to one of claims 1 to 4, wherein Soya oil, castor oil, sunflower oil, wheat germ oil, macadamia nut oil are used as oil-soluble compounds in preparation B.

6. Use of a cosmetic and/or dermatological composition according to one of the previous claims for cleaning the skin and/or the cutaneous appendages.

7. Use of a cosmetic and/or dermatological composition according to one of the previous claims as a shower gel, bath gel or shampoo.

## Revendications

1. Un produit cosmétique ou dermatologique contenant
a) un produit de nettoyage cosmétique ou dermatologique ou les deux à la fois A en quantité de 25 à 75 en pourcentage du poids de la préparation totale,
b) une préparation transparente B faite à partir d'une ou de plusieurs combinaisons huileuses et d'au moins un agent détersif dans une quantité de 25 à 75 en pourcentage du poids de la préparation totale, **caractérisée par le fait que** les deux préparations A et B sont gardées dans des chambres séparées dans un contenant-emballage commun et sont utilisées à partir de cet emballage. Les deux préparations sont tirées parallèlement de cet contenant soit à travers une ouverture, les deux préparations étant mélangées avant de sortir de l'ouverture, ou elles sortent dans l'ouverture sous la forme d'un ruban qui contient la préparation totale, ou à travers deux ouvertures séparées. Ce qui aussi la caractérise c'est **le fait que** dans la préparation B un ou deux agents détersifs huileux sont utilisés dans la concentration totale de 1 à 10 en pourcentage du poids total de la préparation B.

2. Un produit cosmétique ou dermatologique ou les deux à la fois selon la revendication 1, **caractérisé par le fait que** l'on emploie comme agents détersifs huileux dans la préparation B, du TIPA- sulfate de laureth, de la cocamide DEA et du PEG-40 perisostearat de Sorbitan.

3. Un produit cosmétique ou dermatologique ou les deux à la fois selon une des exigences 1 à 12, **caractérisé par le fait que** la préparation A contient des agents détersifs anioniques, cationiques, amphotères ou avec à la fois toutes les caractéristiques dans une concentration totale de 1 à 30 en pourcentage du poids de la préparation totale de la préparation A.

4. Un produit cosmétique ou dermatologique ou les deux à la fois selon une des exigences 1 à 3, **caractérisé par le fait que** l'on utilise dans la préparation A du sodium laureth sulfate, du sodium myreth sulfate, de la cocamide propyl bétaine, du lauryl glucoside, du sodium cocoyl glutamat ou du sodium coco amphoceat ou tous ensembles.

5. Un produit cosmétique ou dermatologique ou les deux à la fois selon une des exigences de 1 à 4, **caractérisé par le fait que** l'on utilise comme combinaisons huileuses dans la préparation B, de l'huile de soja, de l'huile de ricin" de l'huile de tournesol, de l'huile de germe de blé, de l'huile de macadamia.

6. Utilisation d'un produit cosmétique ou dermatologique ou des deux à la fois selon une des exigences sus-citées pour le nettoyage de la peau ou des formations appendices de la peau.

7. Utilisation d'un produit cosmétique ou dermatologique ou des deux à la fois comme gel de douche, de bain ou de nettoyant pour cheveux selon une des revendications sus-citées.
